# EUROPEAN PATENT APPLICATION

(11) **EP 0 972 847 A2**
(43) Date of publication of application: **19.01.2000**
(21) Application number: 98401007.4
(22) Date of filing: 24.04.1998
(51) Int. Cl.: C12Q 1/68, C12Q 1/18

(54) **Method for screening antimycotic substances**

(71) Applicant: HOECHST MARION ROUSSEL, 92800 Puteaux (FR)
(72) Inventor: Diu-Hercend, Anita, 94220 Charenton Le Pont (FR); Entian, Karl-Dieter, 61440 Oberursel (DE); Koetter, Peter, 61440 Oberursel (DE)
(74) Representative: Archimbeaud, Nathalie Christiane

(57) **Abstract**

The present invention concerns a method for the screening of antimycotic substances wherein an essential gene from mycetes or a functionally similar mycete gene, or the corresponding encoded protein, is used as target.

## Description

The present invention relates to a method for screening for antimycotic substances in which essential genes from mycetes, particularly from Saccaromyces cerevisiae (S.cerevisiae) as well as functionally similar genes from other mycetes, or the corresponding encoded proteins, are used as targets.

The spectrum of known fungal infections stretches from fungal attack of skin or nails to potentially hazardous mycotic infections of the inner organs; Such infections and resulting diseases are known as mycosis.

Antimycotic substances (fungistatic or fungicidal) are used for treatment of mycosis. However, up to now, relatively few substances with pharmacological effects are known, such as Amphotericin B, Nystatin, Pimaricin, Griseofulvin, Clotrimazole, 5-fluoro-cytosine and Batraphene. The drug treatment of fungal infections is extremely difficult, in particular because both the host cells and the mycetes, are eucaryotic cells. Administration of drugs based on known antimycotic substances results therefore often in undesired side-effects, for example Amphotericin B has a nephrotoxic effect. Therefore, there is a strong need for pharmacologically efficient substances usable for the preparation of drugs, which are suitable for prophylactic treatments of immunodepressive states or for the treatment of an existing fungal infection. Furthermore, the substances should exhibit a specific spectrum of action in order to selectively inhibit the growth and proliferation of mycetes without affecting the treated host organism.

The aim of the present invention is to provide a method for the identification of antimycotic substances. An essential feature of this method is that essential genes from mycetes are used as targets for the screening. The present invention thus concerns a method for screening antimycotic substances wherein an essential gene from mycetes or a functionally similar gene in another mycete, or the corresponding encoded protein, is used as target and wherein the essential gene is selected from the group consisting of YLR186w, YLR215c, YLR222c, YLR243w, YLR272c, YLR275w, YLR276c, YLR317w, YLR359w, YLR373c, YLR424w, YLR437c, YLR440c, YML023c, YML049c, YML077w, YML093w, YML114c, YML127w, YMR032w, YMR093w, YMR131c, YMR185w, YMR212c, YMR213w, YMR218c, YMR281w, YMR288w, YMR290c, YMR049c, YMR134w, YDR196c, YDR299w, YDR365c, YDR396w, YDR407c, YDR416w, YDR449c, YDR472w, YDR499w, YDR141c, YDR324c, YDR325w, YDR398w, YDR246w, YDR236c, YDR361c, YDR367w, YDR339c, YDR413c, YDR429c, YDR468c, YDR489w, YDR527w, YDR288w, YDR201w, and YDR434w.

According to one embodiment of the method of the invention mycete cells which express the essential gene, or a functionally similar mycete gene, to a different level are incubated with the substance to be tested and the growth inhibiting effect of the substance is determined.

According to another embodiment, said target gene or the corresponding target gene encoded protein is contacted in vitro with the substance to be tested and the effect of the substance on the target is determined.

According to another embodiment, the screened substances inhibit partially or totally the functional expression of the essential genes or the functional activity of the encoded proteins.

According to another embodiment, the mycete species are selected from the group comprising Basidiomycetes, Ascomycetes and Hyphomycetes.

According to another embodiment of the method of the invention said functional similar genes are essential genes from Candida Spp., preferably Candida albicans, or from Aspergillus Spp., preferably from Aspergillus fumigatus.

According to a further embodiment of the above method said mycete cells are haploid S.cerevisiae cells.

According to a particular embodiment of the method of the invention the essential genes of S.cerevisiae are identified by integrating by homologous recombination a selection marker at the locus of the gene to be studied.

The present invention also concerns a method as described above wherein the functionally similar genes are identified by:
a)providing a S.cerevisiae mutant strain in which the gene of S.cerevisiae to be investigated is either integrative or extrachromosomal under the control of a regulated promoter,
b)culturing said mutant strain under growth conditions in which the regulated promoter is active,
c)transforming the mutant strain with a cDNA or genomic DNA that has been prepared from the heterologous mycete-species and that has been integrated into an appropriate vector,
d)altering the culture condition, so that the regulated promoter is switched off and only S.cerevisiae cells which contain a functionally similar gene can survive,
e)isolating and analyzing the cDNA or genomic DNA.

The invention thus discloses that in a first step, essential genes from S.cerevisiae are identified. The invention also discloses that, essential genes from other mycetes are identified starting from the identified essential genes in S.cerevisiae. In order to identify essential genes of S.cerevisiae, individual genomic genes are eliminated through homologous recombination. If the DNA segment thus eliminated concerns an essential gene, then the deletion is lethal for the S.cerevisiae cells in haploid form.

A method, wherein the studied S.cerevisiae gene is replaced by a marker gene can be used to generate the corresponding genomic deletion of S.cerevisiae and to determine the S.cerevisiae cells containing the deletion.

As a selection marker a dominant selection marker (e.g. kanamycin resistance gene) or an auxotrophic marker can for example be used. As an auxotrophic marker, it is possible to use genes coding for key enzymes of amino acid or nucleic base synthesis. For example, one can use as a selection marker the following genes from S.cerevisiae : gene encoding for the metabolic pathway of leucine (e.g.LEU2-gene), histidine (e.g. HIS3-gene) or tryptophan (e.g. TRP-1 gene) or for the nucleic base metabolism of uracil (e.g. URA3-gene).

Auxotrophic S.cerevisiae strains can be used. These auxotrophic strains can only grow on nutritive media containing the corresponding amino acids or nucleotide bases. All laboratory S.cerevisiae strains, containing auxotrophic markers can for instance be used. When diploid S.cerevisiae strains are used, then the corresponding marker gene must be homozygously mutated. Strain CEN.PK2 or isogenic derivates thereof can be used.

Strains containing no suitable auxotrophic marker can also be used such as prototrophic S.cerevisiae strains. Then a dominant selection marker e.g. resistance gene, such as kanamycin resistance gene can be used. A loxP-KanMX-loxP cassette can advantageously be used for this purpose.

For the homologous recombination replacing the whole DNA sequence or part thereof of a S.cerevisiae gene, DNA fragments are used wherein the marker gene is flanked at the 5'- and 3'-ends by sequences which are homologous to the 5'- and 3'-ends of the studied S.cerevisiae gene.

Different processes can be used for the preparation of the corresponding DNA fragments which are also appropriate for the deletion of any specific S.cerevisiae gene. A linear DNA-fragment is used for the transformation of the suitable S.cerevisiae strain. This fragment is integrated into the S.cerevisiae genome by homologous recombination. These processes include:
1. "Conventional method" for the preparation of deletion cassettes (Rothstein, R.J. (1983) Methods in Enzymology, Vol. 101, 202-211).
2. "Conventional Method" using the PCR technique ("modified conventional method")
3. SFH (short flanking homology)- PCR method (Wach, A. et al. (1994) Yeast 10: 1793-1808; Gültner, U. et al. (1996) Nucleic Acids Research 24:2519-2524).

1. In the "conventional method" for the preparation of deletion cassettes in the S.cerevisiae genome, the gene to be studied is either already present in an appropriate vector or is integrated in such a vector. With this method, any pBR- pUC- and pBluescript®-derivates can be used for example. A major part of the target gene sequence is eliminated from the vector, for instance using appropiate restriction sites, conserving however the 3'- and 5'-regions of the studied gene inside the vector. The selected marker gene is integrated between the remaining regions.
2. In the modified form of this "conventional method", PCR is used. This method allows amplification of the 3'- and 5'-terminal regions of the coding sequence of the studied S.cerevisiae gene. This method amplifies selectively both terminal regions of the studied gene, therefore, two PCR-reactions must be carried out for each studied gene, amplifying once the 5'-end, and once the 3'-end of the gene. The length of the amplified terminal DNA-fragment depends among others of the existing restriction sites. The amplified terminal ends of the studied gene have generally a length of 50 to 5000 base pairs (bp), preferably a length comprised between 500 and 1000 bp.
   As template for the PCR-reactions, genomic DNA of S.cerevisiae or wild-type genes can be used. The primer-pairs (a sense and an antisense primer, respectively) are constructed so that they correspond to the 3' -end and the 5'-end sequence of the studied S.cerevisiae gene. Especially, the primer is selected such as to allow its integration by way of appropriate restriction sites.
   As vectors, pBR- pUC- and pBluescript®-derivates can be used. In particular vectors already containing a gene encoding for the selection marker, are appropriate. In particular, vectors can be used, which contain genes of the selection marker HIS3, LEU2, TRP1 or URA3.
   The DNA segments of the studied S.cerevisiae gene, obtained by PCR, are integrated in the vector at both sides of the selection marker, so that subsequently, as in the "conventional method", the selection marker is flanked on both ends by DNA sequences which are homologous to the studied gene.
3. Homologous recombination in S.cerevisiae takes place in a very efficient and precise manner and the length of the DNA sequence homologous to the studied S.cerevisiae gene flanking the selection marker gene can in fact be considerably shorter than with the "modified conventional method". The flanking ends homologous to the studied S.cerevisiae gene need to present a length of only about 20-60 bp, preferably 30-45 bp. The SFH-PCR method is particularly advantageous inasmuch as the laborious cloning step can be obviated.

A PCR reaction is carried out on a DNA-template containing the gene for the selection marker to be used, wherein the primers are constructed so that the DNA sequence of the sense primer is homologous to the 5'-end of the selection marker sequence and so that the primer presents in addition at its 5'-end a region of preferably 40 nucleotides, which corresponds to the 5'-terminal sequence of the studied S.cerevisiae gene. The antisense primer is constructed in an analogous manner, i.e. it is complementary to the 3'-end of the gene sequence of the selection marker, wherein this primer contains at its 5'-end a region of also preferably 40 nucleotides, which corresponds to the complementary strand of the 3'-terminal coding sequence of the studied gene.

For the amplification of S.cerevisiae genes to be studied by SFH-PCR method, vectors containing the gene for the auxotrophic marker or selection marker can be used. Especially, plasmid pUG6 is used as the template. This plasmid contains a loxP-KanMX-loxP cassette (Gültner, U. et al. (1996) Nucleic Acids Research 24: 2519-2524). In other terms, the Kanamycin resistance gene is flanked at both ends by a loxP sequence (loxP-KanMX-loxP cassette). This cassette is advantageous in that the Kanamycin resistance gene can be eventually eliminated from the S.cerevisiae genome after integration of the loxP-KanMX-loxP cassette into the S.cerevisiae gene to be studied. Cre-recombinase of bacteriophage P1 can be used for this purpose. Cre-recombinase recognizes the loxP sequences and induces elimination of the DNA located between the two loxP sequences by a homologous recombination process. As a result only one loxP sequence remains and the so-called marker regeneration occurs, i.e. the S.cerevisiae strain may be transformed again using the loxP-KanMX-loxP cassette. This is particularly advantageous, when at least two functionally similar genes are to be deleted in order to obtain a lethal phenotype.

With the PCR-method, the PCR reaction primers are at the 3'-end a preferably 20 nucleotide long sequence, which is homologous to the sequence situated left and/or right of the loxP-KanMX-loxP cassette, and at the 5'-end a preferably 40 nucleotide long sequence, which is homologous to the terminal ends of the gene to be studied.

Using the three methods, one obtains linear deletion cassettes containing the gene encoding the selection marker, which is flanked on both sides by homologous sequences of the gene to be studied. The deletion cassettes are used for the transformation of diploid S.cerevisiae strains. The diploid strain S.cerevisiae CEN.PK2 (Scientific Research & Development GmbH, Oberursel) can be used for example for this purpose. [CEN.PK2 Mata/MAT α ura3-52/ura3-52 leu2-3, 112/leu2-3, 112his3Δ1/his3Δ1 trp1-289/trp1-289 MAL2-8^{c}/MAL2-8^{c} SUC2/SUC2]

The strain CEN.PK2 is prepared and cultivated using known methods (Gietz, R.D. et al. (1992) Nucleic Acids Research 8: 1425; Güldener, U. et al. (1996) Nucleic Acids Research 24:2519-2524).

The cells of the S.cerevisiae strain used are transformed according to known processes with an appropriate DNA quantity of the linear deletion cassette (e.g. Sambrook et al. 1989) . Thereafter, the medium in which the cells are cultivated is replaced by a new medium, a so-called selective medium, which does not contain the corresponding amino acid (e. g. histidine, leucine or tryptophan) or nucleic base (e. g. uracil) or, when using a deletion cassette containing the kanamycin resistance gene, by a medium containing geneticin (G418®) (e.g. a complete medium (YEPD) containing geneticin). Alternatively, the transformed cells may be plated on agar plates prepared using the corresponding media. Thereby, one selects the transformed cells, in which a homologous recombination occured, since only those cells can grow under these modified conditions.

However, in most cases, only one of the two copies of the gene in the double chromosome set of a diploid S.cerevisiae strain is replaced by the DNA of the deletion cassette during the transformation, resulting in a heterozygote-diploid S.cerevisiae mutant strain, wherein one copy of the gene studied is replaced by a selection marker, while the other copy of the gene is maintained in the genome. This presents the advantage that in case of a deletion of an essential gene, due to the existence of the second copy of the essential gene, the mutant S.cerevisiae strain is still viable.

The proper integration of the deletion cassette DNA at the predetermined chromosomal gene locus (gene locus of the gene to be studied) may be checked by Southern-Blot Analysis (Southern, E.M. (1975) J. Mol. Biol. 98:503-517) or by diagnostic PCR analysis using specific primers (Güldener, U. et al. (1996) Nucleic Acids Research 24:2519-2524)

The genetic separation of individual diploid cells may be monitored by tetrad analysis. To this end, reduction division (meiosis) is induced in the diploid cells, especially heterozygote mutant strains, using known methods such as nitrogen impoverishment on potassium acetate plates (Sherman, F. et al. (1986) Cold Spring Harbor Laboratory Press, Cold Spring Harbor, N. Y.; Guthrie, C. and Fink, G.R. (1991) Methods in Enzymology, Vol 194. Academic Press, San Diego, 3-21; Ausubel, F. M. et al. (1987) Current Protocol in Molecular Biology John Wiley and Sons, Inc., Chapter 13). Meiosis results only in asci with four ascospores (segregated) , which can be indivualized after partial enzymatic digestion of the ascospore wall with zygmolyase (Ausubel et al. (1987)) by way of micromanipulators (e.g. SINGER). For example when a tetrad analysis is carried out on a heterozygote-diploid mutant strain in which an essential gene present in the double chromosome set is replaced by homologous recombination, then only two segregated ascospores are viable, namely those which carry the essential gene. The two remaining segregated ascospores are not viable because they lack the essential gene.

In order to check if the genes studied by this method are really essential or if the homologous recombination leads to an alteration of an essential gene adjacent to the gene locus of the gene studied, the heterozygote diploid S.cerevisiae mutant strain is transformed with a centromere plasmid containing said studied gene.

A tetrad analysis is carried out on the transformants. When four instead of two viable segregates are obtained, then the studied gene contained in the centromere plasmid can complement the defect of the two non-viable haploid S.cerevisiae cells/mutant strains, which demonstrates that the studied S.cerevisiae gene is essential.

Preferably, plasmids present in low copy number, e.g. one or two copies per cell are used as centromere plasmids. For example plasmids pRS313, pRS314, pRS315 and pRS316 (Sijkorski, R. S. and Hieter, p. (1989) Genetics 122: 19-27) or similar plasmids can be used for this purpose. Preferably, the studied genes are integrated in said plasmids including their 3'- and 5'-end non-coding regions.

Individual S.cerevisiae genes may be studied using the above-described method, their sequences being totally or partially known. The complete genomic sequence of S.cerevisiae was made accessible to the public via the WWW (World Wide Web) on April 24, 1996.

Different possibilities exist to have access to the S.cerevisiae genomic DNA sequence via the WWW.

MIPS (Munich information Centre of Protein Sequence) Address http://speedy.mips.biochem.mpg.de/mips /yeast/

SGD (Saccharomyces Genome Database, Stanford) Address http: //genome-www. stanford. edu/Saccharomyces

YPD(Yeast Protein Database, Cold Spring Harbor)

Address http://www.proteome.com/YPDhome.html

The complete S.cerevisiae DNA sequence is also accessible via FTP (file transfer protocol) in Europe (e.g. at the address: ftp.mips.embnet.org) in the U.S.A. (address: genome-ftp.stanford.edu) or in Japan (address: ftp.nig.ac.jp).

57 essential genomic S.cerevisiae genes have been identified by this way. These essential genes are listed in table 1. Table 1 contains the systematic gene name of the essential genes (corresponding to the denomination under which the corresponding DNA sequences are accessible in databanks), the deleted nucleotides and the corresponding amino acids of the essential genes (position 1 is taken as reference, this latter corresponding to the A of the probable initiation codon ATG of the ORF). Furthermore, the information available concerning the functions of respective genes or of the encoded proteins and/or homologies/similarities to other genes or proteins are indicated.

The data of table 1 emphasize that despite the fact that the S.cerevisiae gene DNA sequences are known, very little is known today about the function, the characteristic properties of these genes, the essential function of these genes, or the proteins encoded by the same.

According to one embodiment of the method, essential genes of S.cerevisiae are used to identify corresponding functionally similar genes in other mycetes.

By functionally similar genes in other mycete species, is meant genes which have a function similar or identical to that of the identified essential genes of S.cerevisiae. Functionally similar genes in other mycetes may, but need not be homologous in sequence to the corresponding essential S.cerevisiae genes. Functionally similar genes in other mycetes may exhibit only moderate sequence homology at the nucleotide level to the corresponding essential S.cerevisiae genes. By moderate sequence homology it is meant in the present invention genes having a sequence identity, at the nucleotide level, of at least 50%, more preferably of at least 60% and most preferably of at least 70%.

In addition, functionally similar genes in other mycetes may, but need not encode proteins homologous in sequence to the proteins encoded by the essential S.cerevisiae genes. Functionally similar proteins in other mycetes may exhibit moderate protein sequence homology to the proteins encoded by the essential S.cerevisiae genes.

By moderate protein sequence homology is meant in the present invention proteins having a sequence identity, at the amino-acid level, of a least 40%, preferably of at least 50%, more preferably of at least 60% and most preferably of at least 70%.

Genes homologous in sequence may be isolated according to known methods, for example via homologous screening (Sambrook, J. et al. (1989) Molecular Cloning Cold Spring Harbor Laboratory Press, N.Y.) or via the PCR technique using specific primers from genomic libraries and/or cDNA libraries of the corresponding mycetes.

According to one embodiment, genes homologous in sequences are isolated from cDNA libraries. In order to find out functionally similar genes in other mycetes, mRNA is isolated from mycete species to be studied according to known methods (Sambrock et al. 1989) and cDNA is synthesized according to known methods (Sambrock et al. 1989; or cDNA synthesis kits, e.g. from STRATAGENE).

The prepared cDNA is directionally integrated in a suitable expression vector.

For example, synthesis of the first cDNA strand may be carried out in the presence of primers having appropriate restriction sites in order to allow a subsequent cloning in the proper orientation with respect to the expression vector promoter. As restriction sites, any known restriction site may be used. As a primer, for instance the following primer, 50 nucleotides long may be used:
5' -GAGAGAGAGAGAGAGAGAGAACTAGTXXXXXXTTTTTTTTTTTTTTTTTT-3'

The sequence (X)₆ represents an appropriate restriction site, for example for XhoI.

After two-strand synthesis, the cohesive ends of the double stranded cDNA are filled (blunt end) and the cDNA ends are then ligated using a suitable DNA adaptor sequence. The DNA adaptor sequence should contain a restriction site which should be different from the restriction site used in the primer for the synthesis of the first cDNA strand. The DNA adaptor may comprise for example complementary 9- or 13-mer oligonucleotides, whose ends represent the cohesive end of a restriction site. These ends may be for example a EcoRI-site:
5' XXXXXGGCACGAG 3'
3' XCCGTGCTC 5'

The single-stranded X in the adaptor sequence represent the cohesive end of a restriction site.

The cDNA provided with corresponding adaptor sequences is then cleaved using restriction endonuclease, whose recognition site was used in the primer for the synthesis of the first cDNA strand, for example XhoI. The cDNA thus obtained would have according to this example 3'-XhoI and 5'-EcoRI protruding ends and could be therefore directionally integrated into an expression vector cleaved with XhoI and EcoRI.

As expression vectors, among others, E. coli/S.cerevisiae shuttle vectors, i.e. vectors usable in E. coli as well as in S.cerevisiae are suitable. Such vectors may then be amplified for instance in E. coli. As expression vectors, those which are present in a high copy number as well as those present in a low copy number in S.cerevisiae cells can be used. For this purpose, for example vectors selected in the group consisting of pRS423 - pRS426 (pRS423, pRS424, pRS425, pRS426) and/or pRS313-pRS316 (pRS313, pRS314, pRS315, pRS316) (Sikorki, R.S. and Hieter, p. (1989) Genetics 122: 19-27; Christianson T. W. et al. (1992) Gene 110: 119-122) are suitable.

Expression vectors should contain appropriate S.cerevisiae promoters and terminators. In case they do not have these elements, the corresponding promoters and terminators are inserted in such a way that a subsequent incorporation of the generated cDNA remains possible. Particularly suitable are the promoters of S.cerevisiae genes MET25, PGK1, TPI1, TDH3, ADHI, URA3. One may use promoters of the wild-type gene in non modified form as well as promoters which were modified in such a way that certain activator sequences and/or repressor sequences were eliminated. As terminators, for example the terminators of the S.cerevisiae genes MET25, PGK1, TPI1, TDH3, ADHI, URA3 are suitable.

According to another embodiment, genes homologous in sequence are isolated from genomic libraries. Genomic DNA libraries from mycetes can be prepared according to procedures known (for example as described in Current Protocols in Molecular Biology, John Wiley and Sons, Inc). For example, genomic DNA from mycetes can be prepared using known methods for yeast cell lysis and isolation of genomic DNA (for example commercially available kits from Bio101, Inc). The genomic DNA can be partially digested using a restriction enzyme such as Sau3AI and the fragments are size-selected by agarose gel electrophoresis. DNA fragments having for example a size of 5-10kb are then purified by classical methods (as for example, using Gene Clean kit from Bio101) and inserted in a E.coli/yeast shuttle vector such as YEP24 (described e.g. by Sanglard D., Kuchler K., Ischer F., Pagani J-L., Monad M. and Bille J., Antimicrobial Agents and Chemotherapy, (1995) Vol.39 No11, P2378-2386) cut by a restriction enzyme giving compatible ends (for example BamHI for Sau3AI-cut genomic DNA). The resulting expression library can be amplified in E.coli. However any known method, appropriate for the preparation of a genomic library, can be used in the present invention.

In order to find the genes in the studied mycete species, which are functionally similar to essential genes of S.cerevisiae, one S.cerevisiae essential gene is placed under control of a regulated promoter, either as an integrative (1) or extrachromosomal (2) gene.
1. For the integration of a regulated promoter in the S.cerevisiae genome, one replaces the native promoter of the selected essential gene by the regulated promoter, for example by homologous recombination via PCR (Güldener et al. (1996). The homologous recombination via PCR can be carried out for example in the diploid S.cerevisiae strain CEN.PK2. The successfull integration into one chromosome can be checked in haploid cells following tetrad analysis.
   Using the tetrad analysis, one obtains four viable ascospores, wherein in two haploid segregates, the selected essential gene is placed under the control of the native promoter, while the essential gene in the two remaining segregates is placed under the control of the regulated promoter.
   The last mentioned haploid segregates are used for the transformation with the cDNA or the genomic DNA present in the recombinant vector.
2. Using the extrachromosomal variant, the selected essential S.cerevisiae gene, is first inserted in a suitable expression vector, for example a E.coli/ S.cerevisiae shuttle vector. For this purpose, the essential gene may be amplified via PCR from genomic S.cerevisiae DNA starting from the ATG initiation codon up to and including the termination codon. The primers used for this purpose may be constructed in such a way that they contain recognition sites for appropriate restriction enzymes, facilitating a subsequent insertion under control of a regulated promoter in an expression vector.

The recombinant expression vector with the plasmid copy of the essential S.cerevisiae gene under the control of a regulated promoter is subsequently used for the transcomplementation of the corresponding mutant allele. The corresponding mutant allele may be selected from the heterozygote-diploid mutant strains prepared by eliminating, partially or totally, by homologous recombination an essential mycete gene listed in table 1 (first column of table 1), as described above.

The expression vector with the selected essential S.cerevisiae gene is transformed in the corresponding heterozygote-diploid mutant strain carrying instead of the selected essential S.cerevisiae gene, a selection marker gene. The transformants are isolated by selection based on the auxotrophic marker contained in the expression vector used. The thus transformed heterozygote-diploid mutant strains are submitted to a tetrad analysis. One obtains four viable segregates. By retracing the corresponding markers of the mutant allele and the expression vector, the transformed wild-type segregates may be distinguished from segregates which do not contain the genomic copy of the essential gene. Segregates, which do not contain the genomic copy of the selected essential gene, are designated as trans-complemented haploid mutant strains. They are subsequently used for transformation with cDNA or genomic DNA libraries from other mycete species present in appropriate vectors.

As regulated promoters, inducible or repressible promoters may be used. These promoters can consist of naturally and/or artificially disposed promoter sequences.

As regulated promoters, for example the promoters of GAL1 gene and the corresponding promoter derivatives, such as for example promoters, whose different UAS (upstream activation sequence) elements have been eliminated (GALS, GALL; Mumberg, J. et al. (1994) Nucleic Acids Research 22:5767-5768) may be used. As regulated promoters, promoters of gluconeogenetic genes may also be used, such as e.g. FBP1, PCK1, ICL1 or parts therefrom, such as e.g. their activation sequence (UAS1 and/or UAS2) or repression sequence (URS, upstream repression sequence) (Niederacher et al. (1992), Curr. Genet. 22: 636-670; Proft et al. (1995) Mol. Gen. Gent. 246: 367-373; Schüller et al. (1992) EMBO J; 11: 107-114; Guarente et al. (1984) Cell 36: 503-511).

A S.cerevisiae mutant strain modified in this manner can be cultivated under growth conditions, in which the regulated promoter is active, so that the essential S.cerevisiae gene is expressed. The S.cerevisiae cells are then transformed with a representative quantity of the library containing the studied mycete species cDNA or genomic DNA. Transformants express additionally the protein whose coding sequence is present in the recombinant vector.

The method contemplates that the growth conditions may be modified in such a way as to inhibit the regulated promoter, under the control of which is the selected essential gene. Especially, growth conditions may be changed by replacing the growth medium. When for example the GAL1 promoter or a derivate thereof is used, one can replace the galactose-containing medium (induced state) by a glucose-containing medium (repressed state).

These modified conditions are lethal for the S.cerevisiae cells in which the recombinant vector does not carry the functionally similar genomic DNA or cDNA of the studied mycete species. On the contrary, the S.cerevisiae cells in which the recombinant vector expresses a functionally similar coding sequence of the studied mycete species, are viable, since in these cells the lethal metabolic defect is complemented by the protein encoded by the functionally similar gene.

The method contemplates that the recombinant vector (the plasmid) is isolated from the surviving transformants using known method (Strathern, J.N. and Higgins, D.R. (1991) . Recovery of plasmids from yeast into Escherichia coli shuttle vectors in:Guthrie, C. and Fink, G.R. Methods in Enzymology, Volume 194. Guide to yeast genetic and molecular Biology. Academic Press, San Diego, 319-329) and the cDNA is analyzed using DNA-analysis methods such as DNA sequencing. (Sanger et al. (1977), Proc. Natl. Acad. Sci. USA 74: 5463-5467)

The method contemplates that essential S.cerevisiae genes may be used for the identification of functionally similar and/or genes homologous in sequence in other mycetes, especially essential genes functionally similar and/or homologous in sequence in mycetes pathogenic to human, animal and plants. For this purpose for example mycetes of the classes phycomycetes or eumycetes may be used, in particular the subclasses basidiomycetes, ascomycetes, especially mehiascomycetales (yeast) and plectascales (mould fungus) and gymnascales (skin and hair fungus) or of the class of hyphomycetes, in particular the subclasses conidiosporales (skin fungus) and thallosporales (budding or gemmiparous fungus), among which particularly the species mucor, rhizopus, coccidioides, paracoccidioides (blastomyces brasiliensis), endomyces (blastomyces), aspergillus, penicilium (scopulariopsis), trichophyton (ctenomyces), epidermophton, microsporon, piedraia, hormodendron, phialophora, sporotrichon, cryptococcus, candida, geotrichum and trichosporon.

Of particular interest is the use of Candida Spp. especially Candida albicans, Aspergillus Spp., especially Aspergillus fumigatus, Coccidioides immitis, Cryptococcus neoformans, Histoplasma capsulatum, Blastomyces dermatitidis, Paracoccidioides brasiliens and Sporothrix schenckii.

The method contemplates that essential mycete genes are used to identify substances which may inhibit partially or totally the functional expression of these essential genes and/or the functional activity of the encoded proteins. Substances may be identified in this fashion, which inhibit mycetes growth and which can be used as antimycotics, for example in the preparation of drugs.

A particular feature of this method is that essential mycete genes or the corresponding encoded proteins are used as targets for the screening of the substances. The method contemplates that essential S.cerevisiae genes as well as functionally similar genes and/or genes homologous in sequence of other mycetes or the corresponding encoded proteins may be used as targets.

According to one embodiment of the screening method of the invention, mycetes cells are provided, which contain the essential gene used as target, and those cells are incubated with the substance to be tested. By this way, the growth inhibitory effect of this substance with respect to the essential target gene is determined.

The mycetes cells which express the essential target gene to a different degree are used, and these cells are then incubated with the substance to be tested and the growth inhibitory effect of this substance is determined.

The method includes the use of two or more mycetes cells, or strains derived therefrom, which differ in that they express the essential target gene to a different degree.

For example, two, three, four, five, ten or more mycetes cells or the corresponding mycetes strains may be comparatively analysed with respect to the growth inhibitory effect of a substance used in a defined concentration. Through such concentration series, antimycotic substances may be distinguished from cytotoxic or inactive substances.

A particular embodiment of the method includes the use of haploid mycetes cells/ strains for the screening, especially haploid S.cerevisiae cells/ strains.

The method contemplates the integration of the essential gene selected as a target in a suitable expression vector.

As expression vectors E.coli/S.cerevisiae shuttle vectors are for example suitable. Especially vectors differing in their copy number per cell may be used. Therefore, one may use vectors, which are present in the transformed S.cerevisiae cells in a high copy number, or one can also use those with a low copy number. One embodiment comprises the use of expression vectors which allow the integration of the target gene in the S.cerevisiae genome.

For example the vectors pRS423, pRS424,pRS425, pRS426, pRS313, pRS314, pRS315, pRS316, pRS303, pRS304, pRS305, pRS306 (Sikorki and Hieter, 1989; Christianson et al. 1992) are appropriate as expression vectors.

The vectors of the series pRS423 - pRS426 are present in a high copy number, about 50 - 100 copies/ cell. On the contrary, the vectors of the series pRS313 - pRS316 are present in a low copy number (1 - 2 copies / cell). When expression vectors from these two series are used, then the target gene is present as an extrachromosomal copy. Using the vector of the series pRS303 - pRS306 allows the integration of the target genes into the genome. Using these three different expression vector types allows a gradual expression of the studied functionally similar essential gene.

The method includes that the growth inhibitory effect of substances with respect to mycetes cells/strains is comparatively determined using expression vectors differing for instance in the copy number of the vector/ cell.

Such cells may express the essential target gene to a different degree and may exhibit a graduated reaction with respect to the substance.

The method includes also, that a target gene expression of different strength is obtained in different mycetes cells (regulated overexpression) by insertion of the target gene in the expression vector between specific selected S.cerevisiae promoters and terminators. S.cerevisiae promoters which are constitutively expressed, but with different strength, are suitable. Examples for such promoters are native promoters of S.cerevisiae genes MET25, PGK1, TPI1, TDH3, ADH1, URA3, TRP1, as well as corresponding derivatives therefrom, for example promoter derivatives without specific activator and/or repressor sequences.

Regulated promoters are also appropriate for the graduated over-expression of the target gene. The native promoters of the GAL1 genes and/or corresponding derivates thereof, for example promoters, in which different UAS elements have been eliminated. (GALS, GALL; Mumberg et al. (1994) Nucleic Acids Research 22: 5767-5768) as well as promoters of gluconeogenetic genes, for example the promoters PBP1, PCK1, ICL1, or parts thereof, for example their activator- (UAS1 or UAS2) or repressor- (URS) sequences are used in corresponding non activable and/or non repressible test promoters (Schüller et al. (1992) EMBO J. 11: 107-114) Guarente et al. (1984) Cell 36: 503-511; Niederacher et al. (1992) Curr. Genet. 22: 363-370; Proft et al. (1995) Mol. Gen. Genet. 246: 367-373).

In the expression vector terminator for example the terminator sequence of S.cerevisiae genes MET25, PGK1, TPI1, TDH3, ADHI, URA3 may be used.

The method includes that by the use of cleverly selected expression vector types and/or the preparation of suitable expression vectors, eventually using promoters of different strength and differently regulated promoters, a series of expression vectors may be constructed, all containing the same target gene, but differing in that they express the target gene to a different extent.

The method includes the transformation of the expression vector in haploid wild-type cells of S.cerevisiae. The thus obtained S.cerevisiae cells/strains are cultivated in liquid medium and incubated in the presence of different concentrations of the tested substance and the effect of this substance on the growth behaviour of the cells/strains expressing the target gene to a different degree is comparatively analysed. The method also includes that haploid S.cerevisiae cells/strains, transformed using the respective expression vector type without target gene, are used as a reference.

The method includes that the screening of the substances can be carried out in different media using regulated promoters, especially GAL1 promoter and its derivates (GALS and GALL), since the expression degree may be largely influenced by the choice of the respective medium. Thus, the expression degree of the GAL1 promoter decreases in the following fashion: 2 % galactose > 1 % galactose + 1 % glucose > 2 % glycerine > 2 % glucose.

The effect of the substances inhibiting the growth of wild-type cells of S.cerevisiae, may be partially or totally compensated by the overexpression of the functionally similar gene of another mycete species.

According to one embodiment, the method for screening antimycotic substances is carried out in vitro by contact of an essential or functionally similar gene or the corresponding encoded protein with the substance to be tested and determination of the effect of the substance on the target. Any in vitro test appropriate for determining the interaction of two molecules, such as a hybridization test or a functional test, can be used (e.g. enzymatic tests which are described in details in Bergmeyer H.U., Methods of Enzymatic Analysis, VCH Publishers). If the screening is carried out using the encoded protein as the target, then the corresponding essential gene is inserted by any suitable method known in the art, such as PCR amplification using a set of primers containing appropriate restriction sites, (Current Protocol in Molecular Biology, John Wiley and Sons, mc) into an expression system, such as E. coli, Baculovirus, or yeast, and the expressed protein is then completely or partially purified by a method known in the art. Any purification method appropriate for the purification of expressed proteins, such as affinity chromatography can be used. If the target protein function is known, a functional test can then be carried out in which the effect of the antimycotic substance on the protein function is determined. If the protein function is unknown, substances which can interact with the target protein, e.g. which bind to the encoded protein, can be tested. In such a case a test such as protection of the target protein from enzymatic digestion by appropriate enzymes can be used.

The method also includes the identification of genes which are functionally similar and/or homologous in sequence to essential S.cerevisiae genes from humans, animals or plants. The corresponding human, animal or plant genes may optionally be used as target genes in the method in order to test if antimycotic substances exhibit an effect on these target genes.

A particular advantage of the method is that in this way substances may be identified which efficiently inhibit mycetes growth and also the influence of these substances on corresponding functionally similar genes and/or genes homologous in sequence to essential S.cerevisiae genes from human, animal or plants may be determined.

The method includes also the possibility to check the existence of functionally similar genes and/or human, animal or plant genes homologous in sequence to the corresponding essential mycete genes, for example by checking homology of the identified essential mycete genes or parts thereof with human, animal or plant sequence genes available in data banks. In this way, it is possible to select at an early stage from the identified essential mycete genes, depending on the aim, those for which no functionally similar gene and/or no human gene homologous in sequence exist, for example.

Thereby, the method offers a plurality of possibilities to identify selectively substances with antimycotic effects, with no harmful effect on human beings.

For example, it is possible to identify substances usable for the preparation of drugs for the treatment of mycosis or prophylaxis in immunodepression states. These substances may be used for example for the manufacture of drugs usable for the treatment of mycotic infections, which occur during diseases like Aids or Diabetes. Substances which may be used for the fabrication of fungicides, especially of fungicides which are harmless for humans and animals, can also be identified.

Furthermore, the method offers the possibility to identify antimycotic substances, which selectively inhibit growth of specific mycete species only.

The screening method is particularly advantageous inasmuch as it is sufficient to know whether the genes are essential, one does not need any additional information regarding the function of the essential genes or the function of the encoded proteins. In addition, it is particularly advantageous for the identification of functionally similar genes to essential S.cerevisiae gene, in other mycetes where the DNA sequence is not available for many of these genes.

### Examples

### Example 1 :

Preparation of a deletion cassette for ORE YML114c, by the classical method using PCR (modified classical method)

### 1)Construction of the plasmids pBluescript®KS+ vector(stratagene; the sequence of which is available on Genbank®X52327) is used as the starting vector for the preparation of the other plasmids.

The vector is cleaved with NotI and the single-stranded ends are subsequently eliminated by incubation with Mung Bean exonuclease. By religation of DNA fragments, the pKS+ΔNotI vector is thus obtained (corresponding to the pBluescript®KS+ without the NotI restriction site).

pKS+ΔNotI is cleaved with PstI and BamHI and the DNA oligonucleotide, synthesized from the pK3/pK4 primer pair described below, is ligated in the opened plasmid. The pKS+neu plasmid thus prepared contains between PstI and BamHI restriction sites, the following novel restriction sites NotI, StuI, SfiI and NcoI (i.e. PstI-NotI-StuI-SfiI-NcoI-BamHI)
5' -GCGGCCGCAAGGCCTCCATGGCCG-3' PK3
5' -GATCCGGCCATGGAGGCCTTGCGGCCGCTGCA-3' PK4

The URA3 gene of S.cerevisiae is amplified via PCR, by use of the primer-pair PK9 and PK10, described below, and an Ycplac33 vector DNA (Gietz, R. D. and Sugino, A. (1988) Gene 74: 527-534) as matrix. The amplified DNA is cleaved with BamHI and NotI and subsequently inserted in pKS+neu which has been cleaved by BamHI and NotI. The plasmid thus obtained is named pPK9/10.

..NotI..
5'-ATCTGCAGCGGCCGCAAACATGAGAATTGGGTAATAACTG-3'   PK9
PstI

..SfiI..
5'-ATGGATCCGGCCATGGAGGCCTTCAAGAATTAGCTTTTCAATTCATC-3'
BamHI PK10

### 2)Preparation of the deletion cassette

The 5'-region of ORF YML114c was amplified by PCR using genomic DNA of S.cerevisiae as template and both primers YML114c-Asp718 and YLM114c-EcoRI, described below.
- YML114c -Asp718:: 5' -GCTGGTACCCGTCGGTCTCTTTACC-3'
- YLM114c-EcoRI:: 5' -TTGGAATTCATTGCCCTTTATGAGTCC-3'

The PCR fragment was subsequently cut with the restriction enzymes Asp718 and EcoRI. The resulting 613BP fragment was inserted in pPK9/10 linearized with Asp718 and EcoRI generating plasmid pYML114c-A.

The 3'region of ORF YML114c was amplified by PCR using genomic DNA of S.cerevisiae as template and both primers YML114c-BamHI and YLM114c-SacI, described below.
- YML114c-BamHI :: 5' -ATCGGATCCGCCAACAATGACAGCG-3'
- YLM114c-SacI :: 5' -GTTGAGCTCTGAGCGTTTGTCCTTG- 3'
The PCR fragment was subsequently cut with BamHI and SacI. The resulting 535bp fragment was inserted in plasmid pYML114c-A linearized with BamHI and SacI generating pYML114c-B. This latter plasmid was used for transformation of S.cerevisiae after linearization with Asp178 and SacI.

### Examples 2-57: Construction of deletion cassettes for the remaining genes listed in table 1

Using the method disclosed in example 1, the deletion cassettes of each of the essential genes can be constructed using as primers those disclosed in table 2.

### Example 58:

S.cerevisiae cells from strain CEN.PK2 are transformed using each about 5 µg DNA of the linear deletion cassette of examples 1 to 57 according to known methods (Gietz et al. 1992; Güldener et al. 1996). The transformation reaction medium is plated on plates on the corresponding selective media. In this manner, the transformants are selected, in which homologous recombination occured, since only these cells can grow under these modified conditions.

The recombinant cells were submitted to a tetrad analysis in the following conditions: Reduction division (meiosis) was induced in the heterozygote mutant strain using known methods (Guthrie C. and Fink,G.R. (1991) Methods in Enzymology, Vol 194, Academic Press, San Diego). The resulting asci were submitted to partial enzymatic digestion with zygmolyase to digest the ascospore wall and separated using a micromanipulator (SINGER Instruments). This analysis demonstrated that all the above-mentioned 57 genes are essential for the growth of S.cerevisiae.

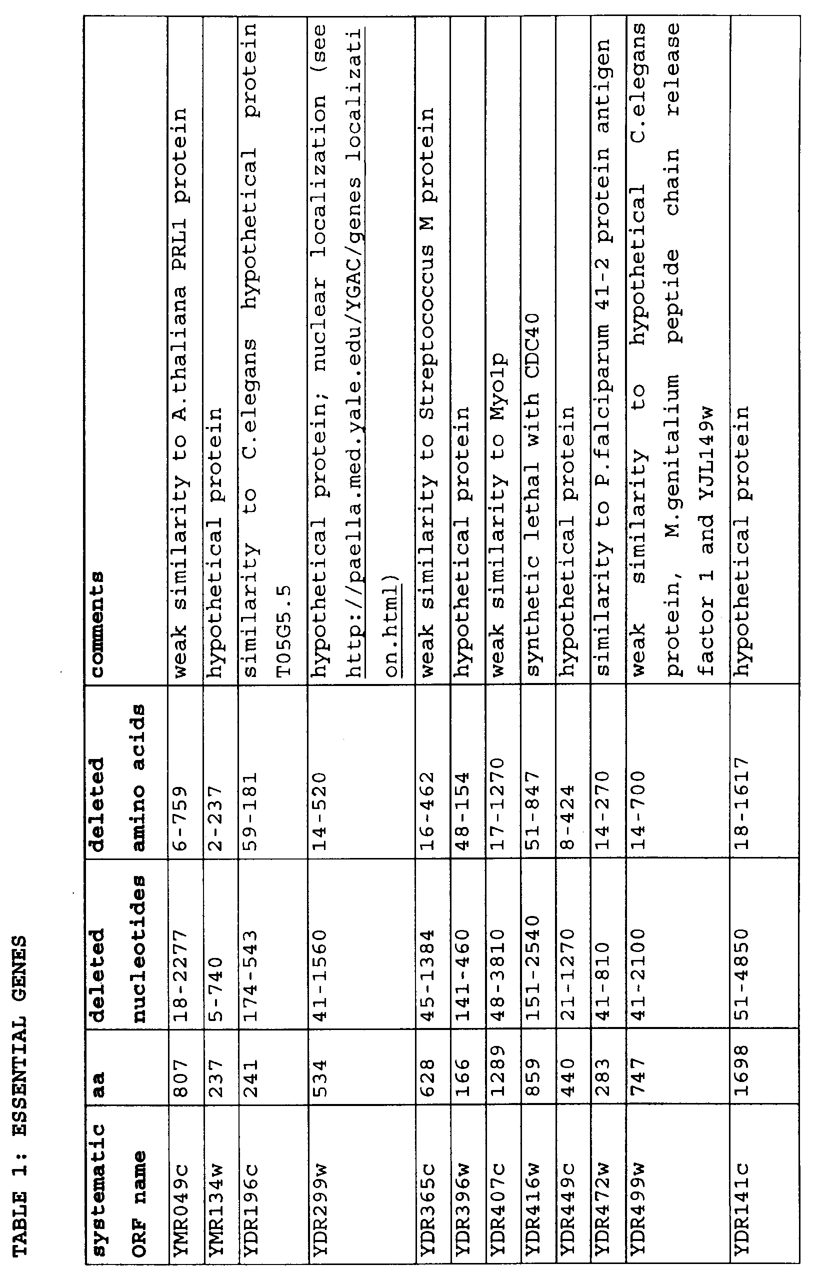

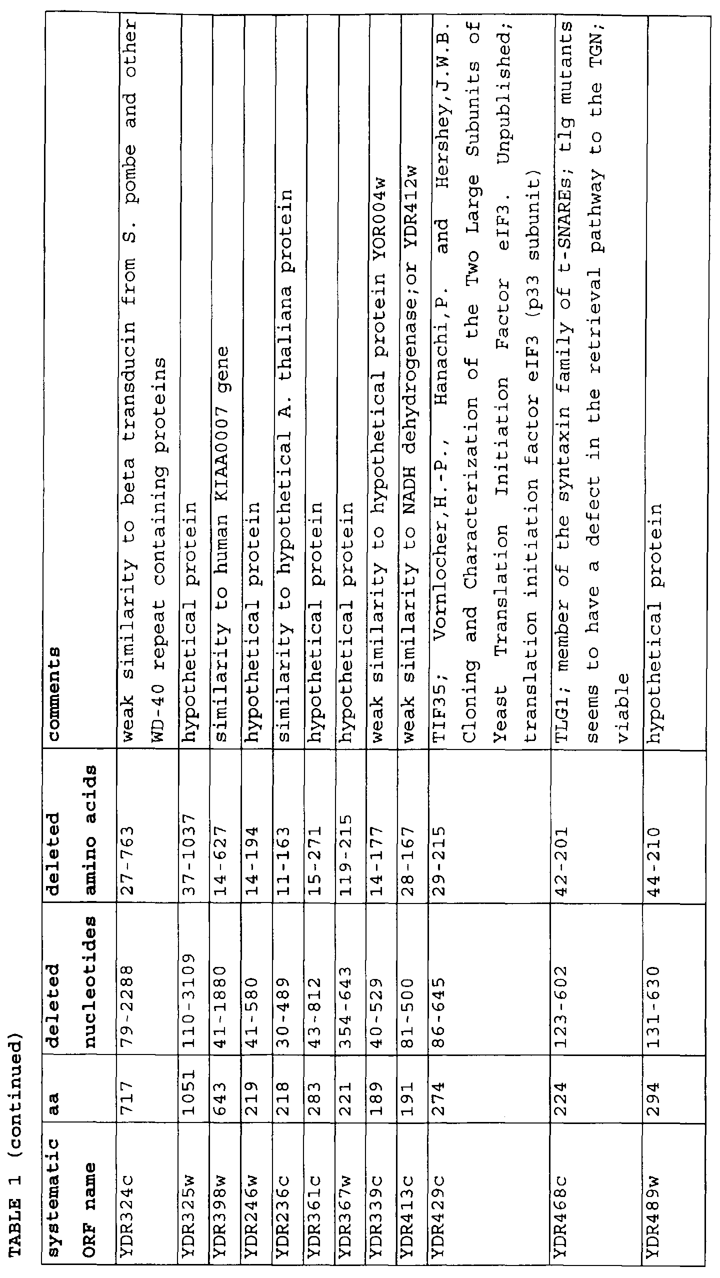

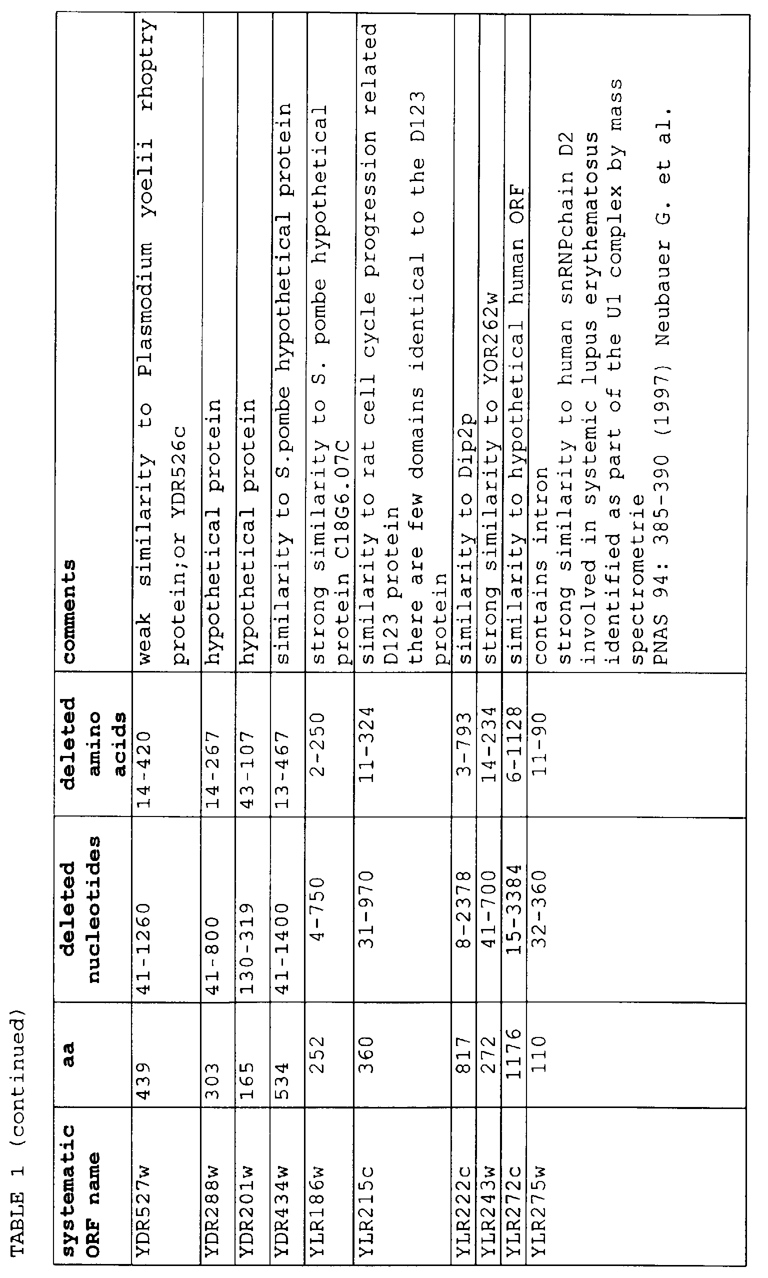

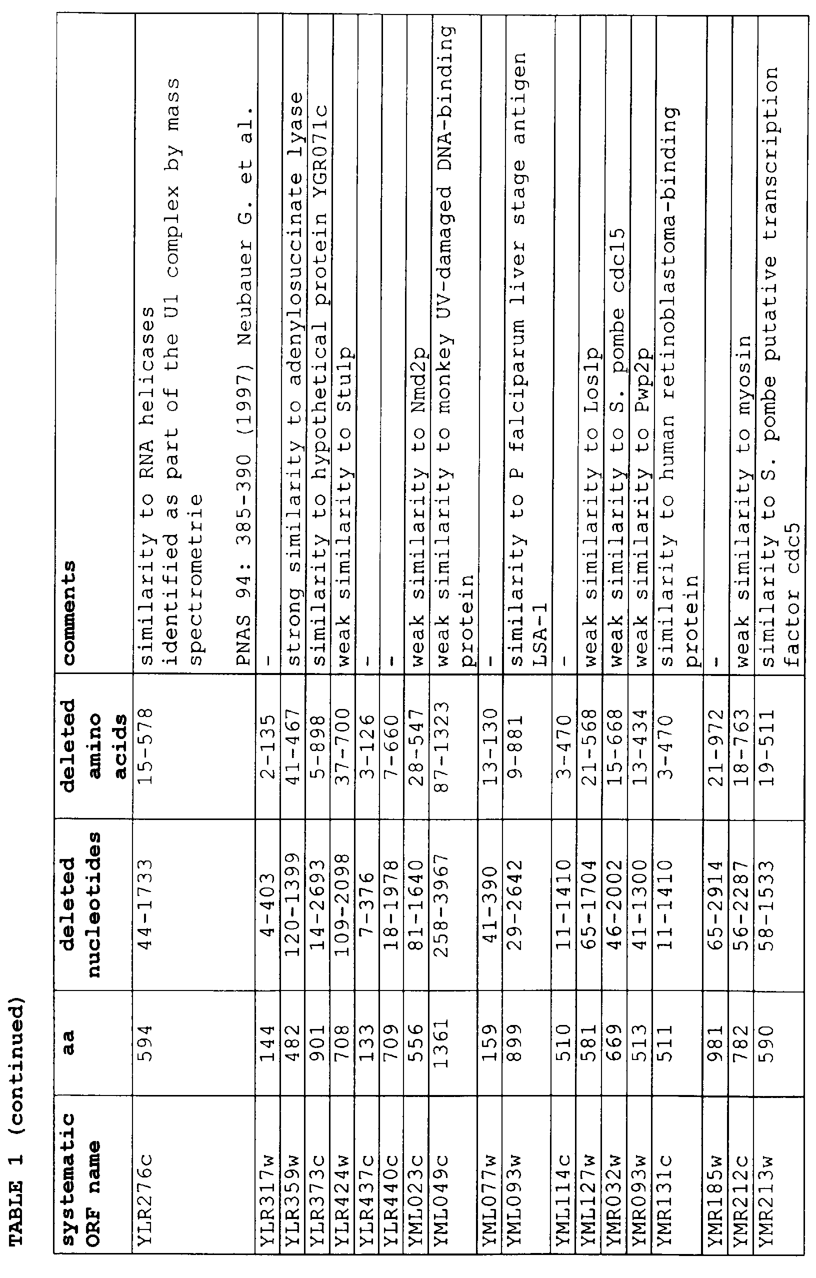

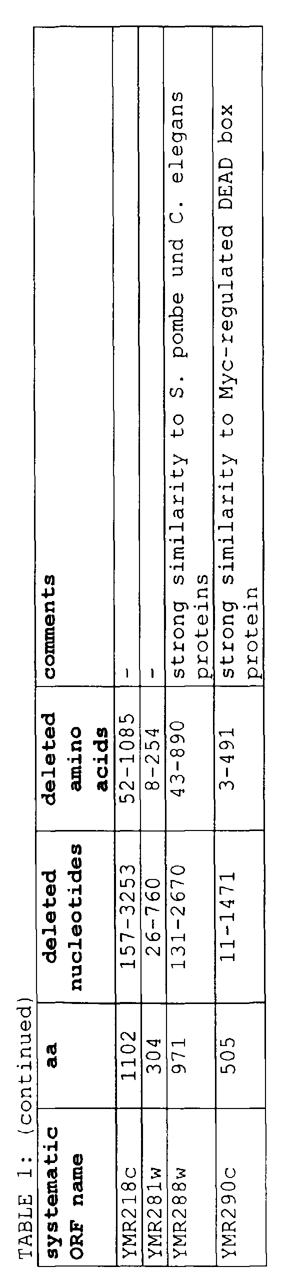

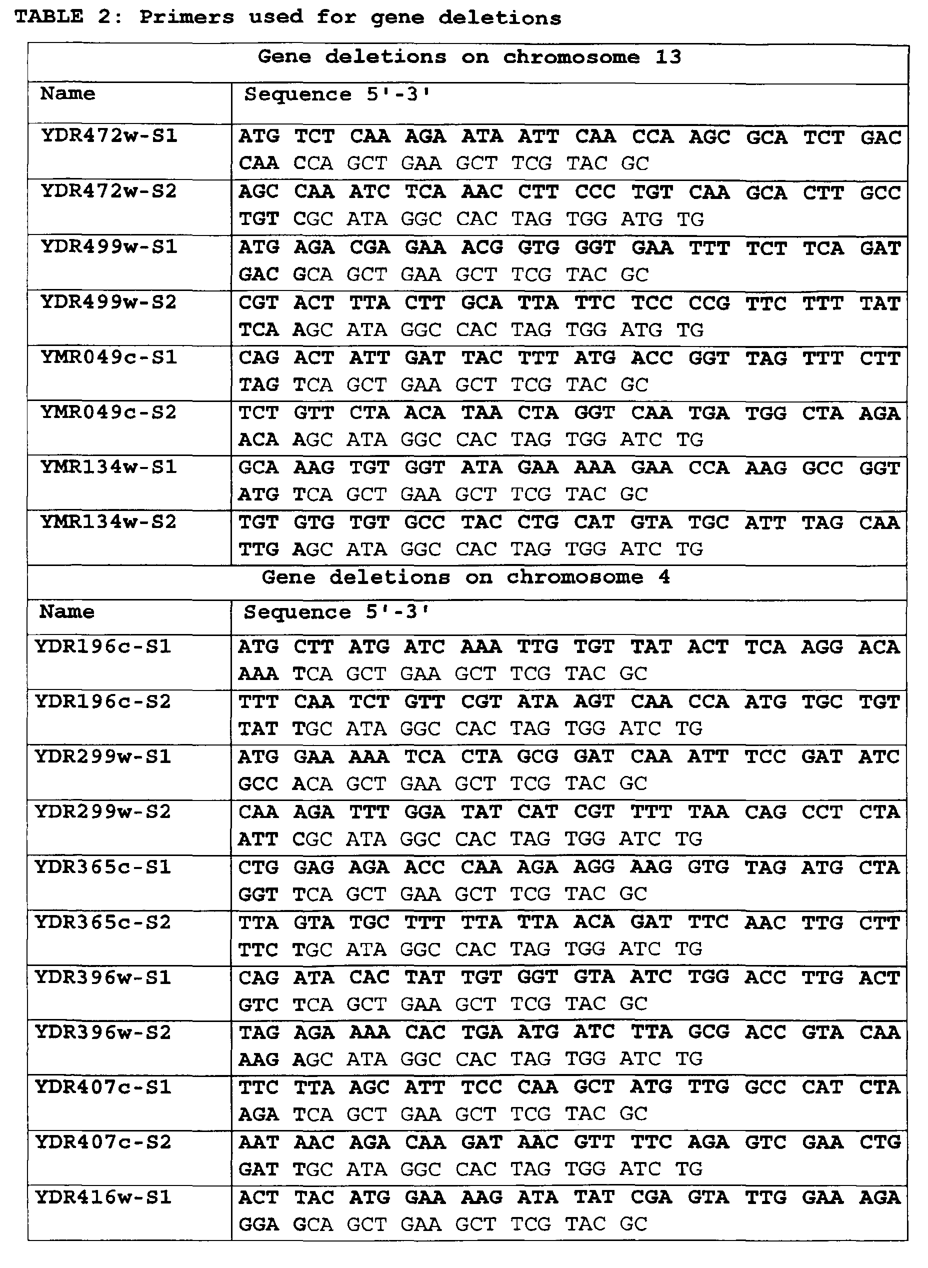

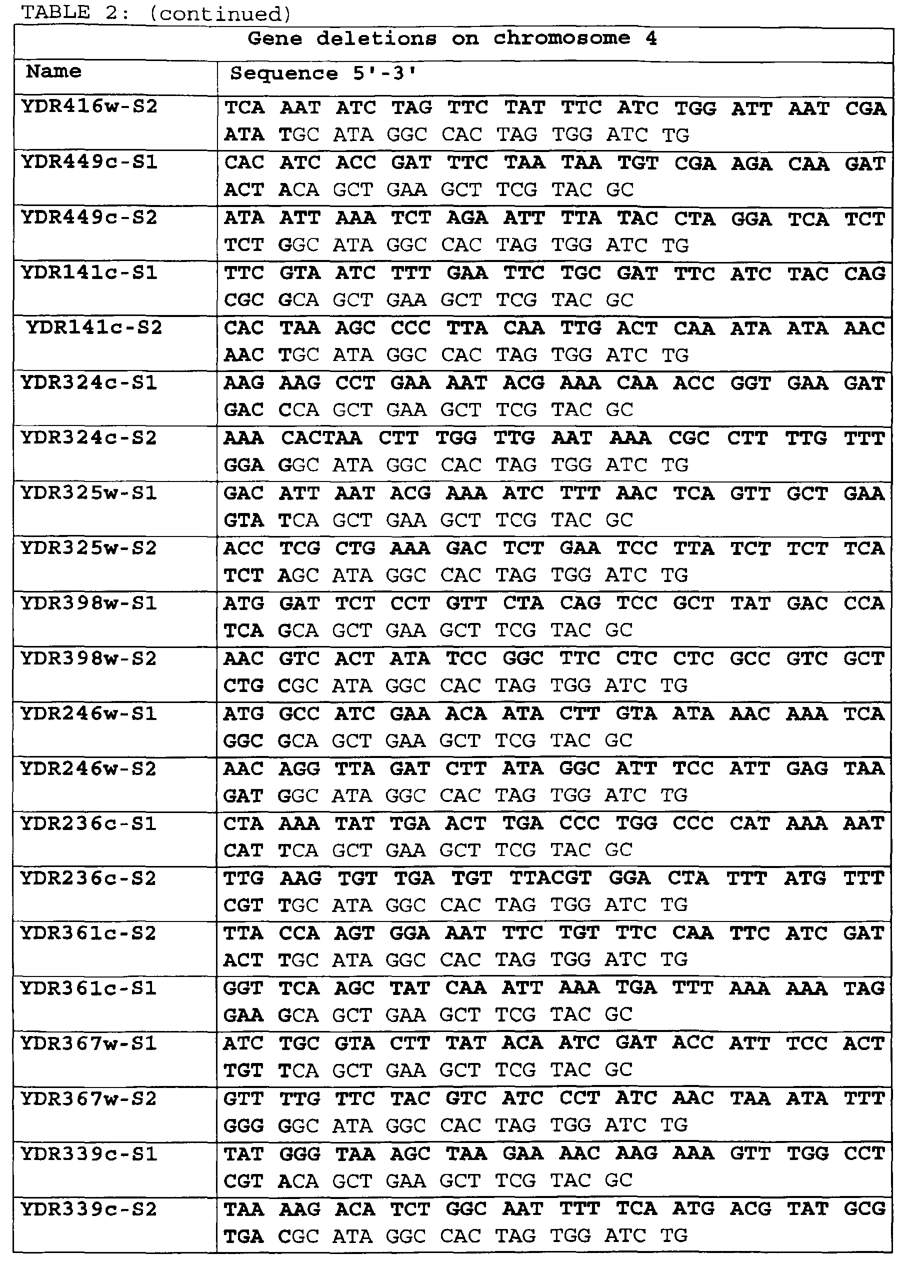

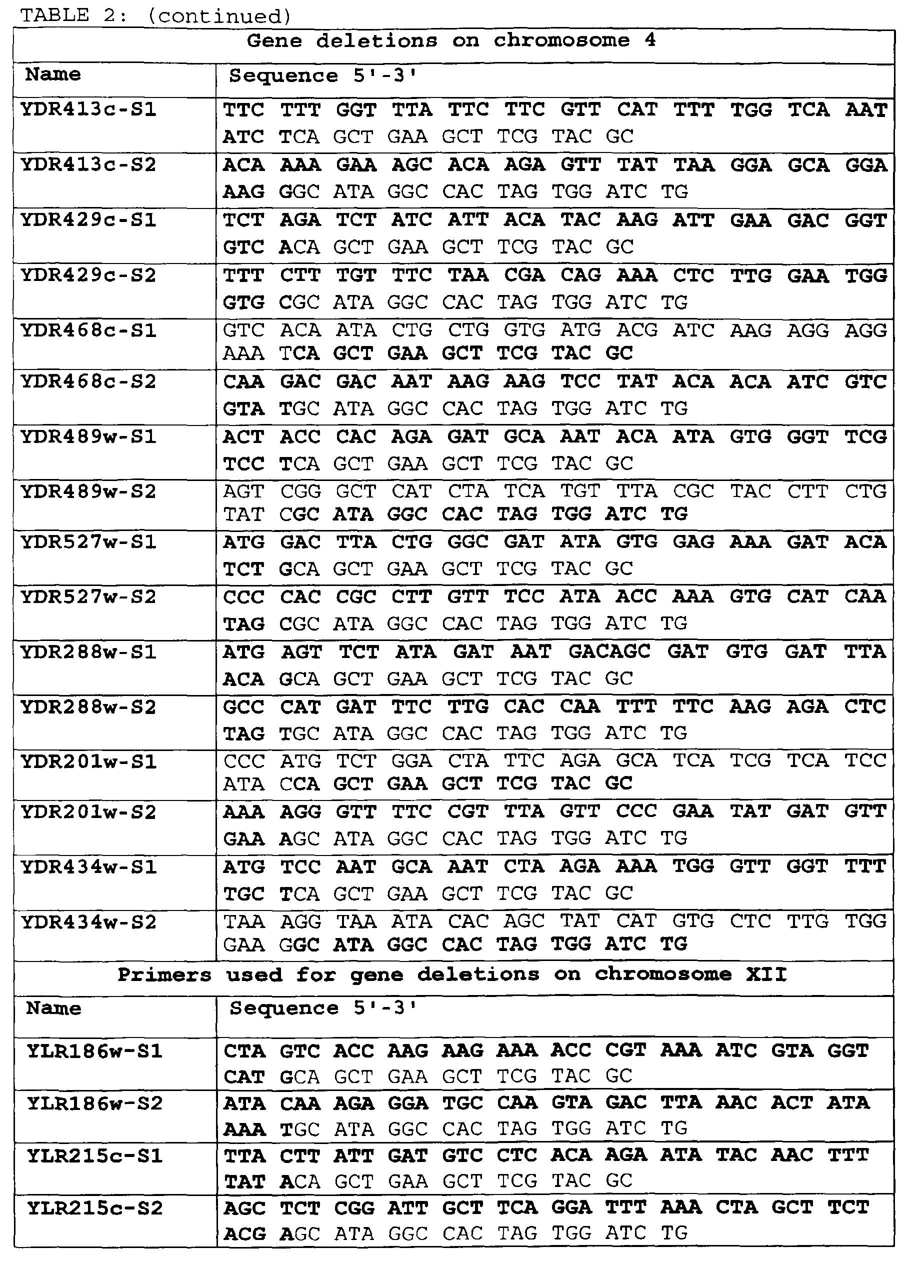

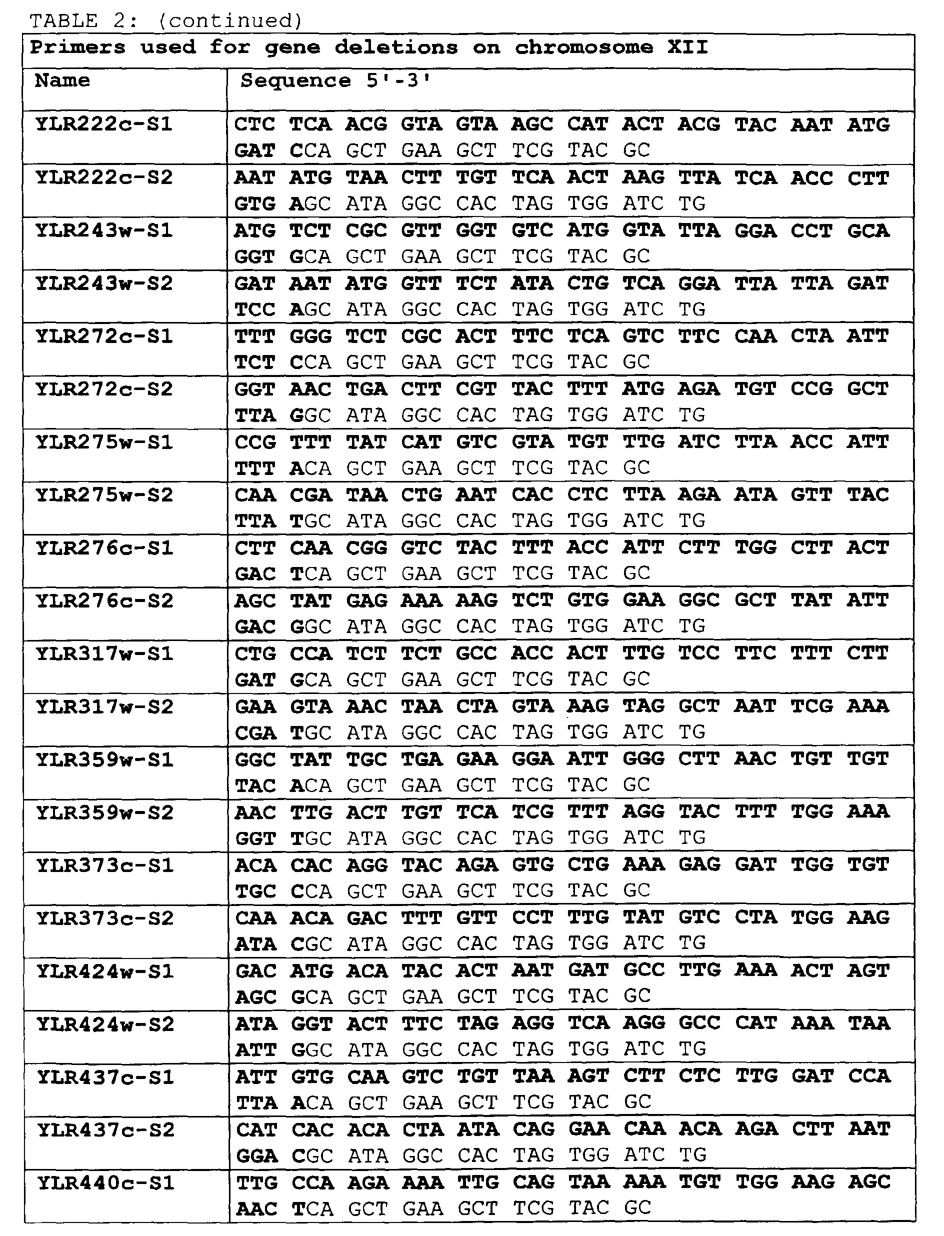

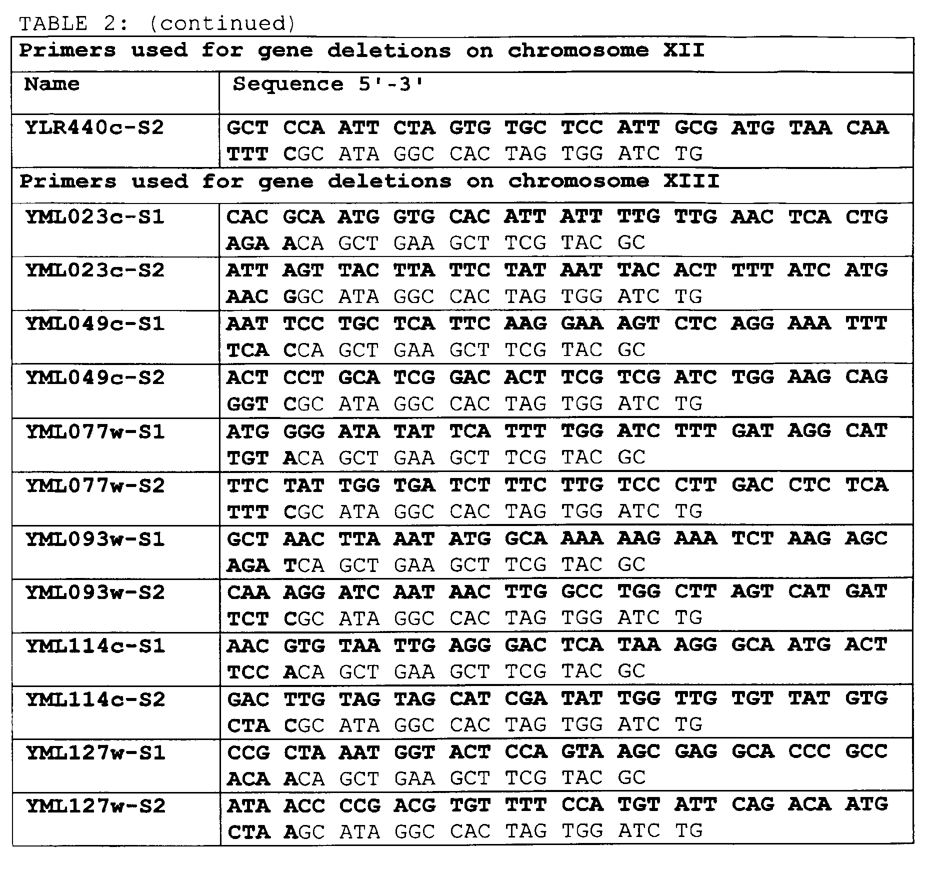

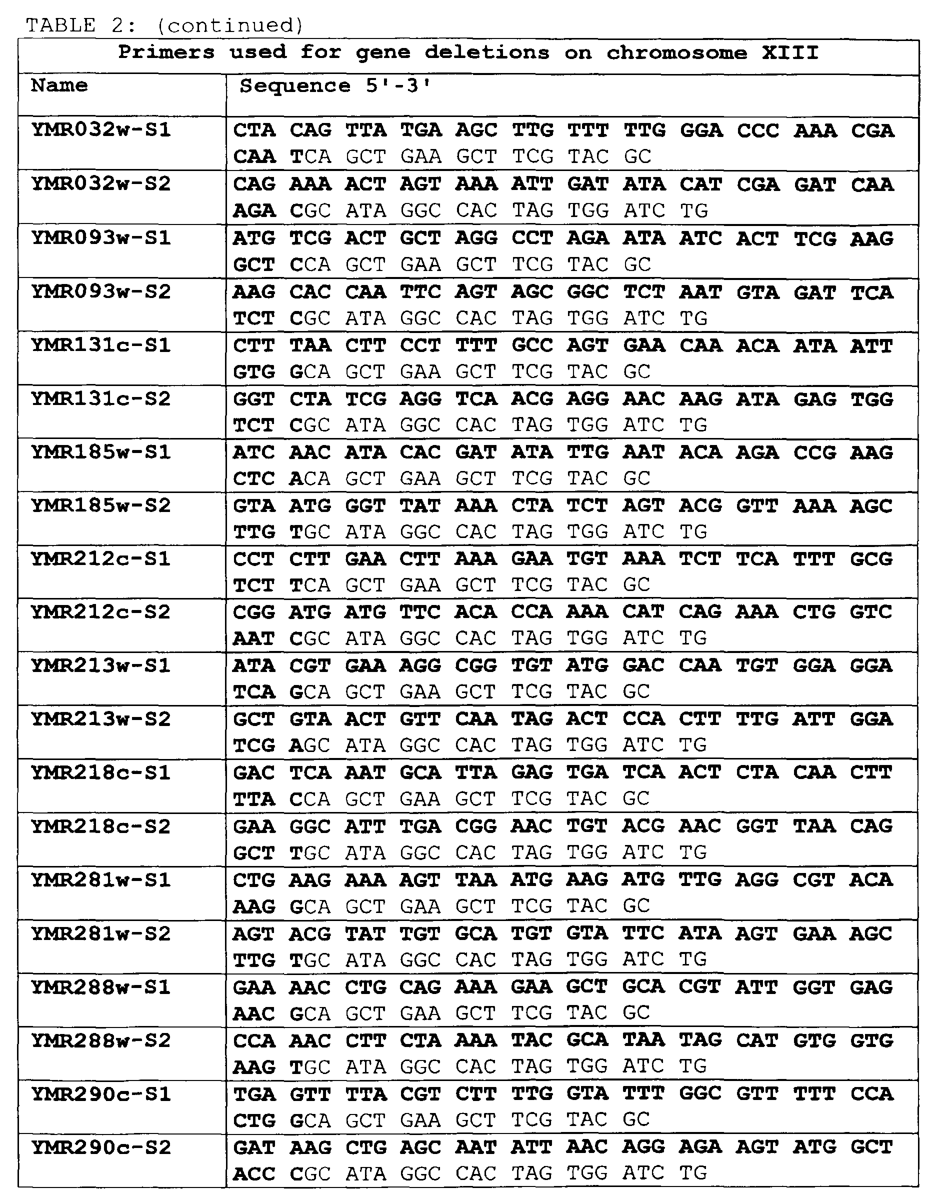

## Claims

1. A method for the screening of antimycotic substances wherein an essential gene from mycetes or a functionally similar mycete gene, or the corresponding encoded protein, is used as target and wherein the essential gene is selected from the group consisting in YLR186w, YLR215c, YLR222c, YLR243w, YLR272c, YLR275w, YLR276c, YLR317w, YLR359w, YLR373c, YLR424w, YLR437c, YLR440c, YML023c, YML049c, YML077w, YML093w, YML114c, YML127w, YMR032w, YMR093w, YMR131c, YMR185w, YMR212c, YMR213w, YMR218c, YMR281w, YMR288w, YMR290c, YMR049c, YMR134w, YDR196c, YDR299w, YDR365c, YDR396w, YDR407c, YDR416w, YDR449c, YDR472w, YDR499w, YDR141c, YDR324c, YDR325w, YDR398w, YDR246w, YDR236c, YDR361c, YDR367w, YDR339c, YDR413c, YDR429c, YDR468c, YDR489w, YDR527w, YDR288w, YDR201w, and YDR434w.

2. The method of claim 1 wherein mycete cells which express the essential gene, or a functionally similar mycete gene, to a different level are incubated with the substance to be tested and the growth inhibiting effect of the substance is determined.

3. The method of claim 1 wherein said target gene or the corresponding target encoded protein is contacted in vitro with the substance to be tested and the effect of the substance on the target is determined.

4. The method according to any one of claims 1-3 wherein the screened substances partially or totally inhibit the functional expression of the essential genes or the functional activity of the encoded proteins.

5. The method according to any one of claims 1-4 wherein the mycete species are selected from the group comprising Basidiomycetes, Ascomycetes and Hyphomycetes.

6. The method according to any one of claims 1-5, wherein said functionally similar genes are essential genes from Candida Spp, or Aspergillus Spp.

7. The method according to claim 6, wherein said functionally similar genes are essential genes from Candida albicans, or Aspergillus fumigatus.

8. The method according to any one of claims 1 to 7 wherein the functionally similar genes are identified by:
a)providing a S.cerevisiae mutant strain in which the gene of S.cerevisiae to be investigated is either integrative or extrachromosomal under the control of a regulated promoter,
b)culturing said mutant strain under growth conditions in which the regulated promoter is active,
c)transforming the mutant strain with cDNA or genomic DNA that has been prepared from the mycete-species to investigate and that has been integrated into an appropriate vector,
d)altering the culture condition, so that the regulated promoter is switched off and only S.cerevisiae cells which contain a functionally similar gene can survive,
e)isolating and analyzing the cDNA or genomic DNA.

9. The method according to claim 8 wherein the functionally similar gene has a sequence identity, at the nucleotide level, with the corresponding S.cerevisiae essential gene of at least 50%, preferably of at least 60%, and most preferably of at least 70%.

10. The method according to claim 8 wherein the functionally similar gene encodes a protein having a sequence identity, at the amino-acid level, with the corresponding S.cerevisiae essential gene encoded protein of at least 40%, preferably of at least 50%, more preferably of at least 60% and most preferably of at least 70%.

11. The method according to any one of claims 1-10 wherein said mycete cells are haploid S.cerevisiae cells.

12. The method according to any one of claims 1-4 or 11 wherein the essential gene of S.cerevisiae are identified by integration through homologous recombination of a selection marker at the locus of the gene to be studied.
